# EUROPÄISCHE PATENTSCHRIFT

(11) **EP 1 601 408 B1**
(45) Veröffentlichungstag und Bekanntmachung des Hinweises auf die Patenterteilung: **03.01.2007**
(21) Anmeldenummer: 04717553.4
(22) Anmeldetag: 05.03.2004
(51) Int. Cl.: A61M 31/00

(54) **ANORDNUNG ZUR FERNGESTEUERTEN FREISETZUNG VON WIRKSTOFFEN**
ARRANGEMENT FOR REMOTE-CONTROLLED RELEASE OF ACTIVE INGREDIENTS
DISPOSITIF POUR LIB RER DES SUBSTANCES ACTIVES DISTANCE

(30) Priorität: 07.03.2003 DE 10310825
(43) Veröffentlichungstag der Anmeldung: 07.12.2005
(73) Patentinhaber: Fachhochschule Jena, 07745 Jena (DE)
(72) Erfinder: ANDRÄ, Wilfried, 07749 Jena (DE); BELLEMANN, Matthias Erich, 07743 Jena (DE); DANAN, Henri, F-67000 Strasbourg (FR); SCHMIEG, Rainer, 99444 Blankenhain (DE)
(74) Vertreter: Bock, Gerhard
(86) Internationale Anmeldenummer: PCT/DE2004/000460
(87) Internationale Veröffentlichungsnummer: WO 2004/078250

(56) Entgegenhaltungen:
- WO-A-01/78836
- DE-A- 2 928 477
- DE-C- 19 745 890
- US-A- 4 239 040
- US-A- 5 279 607

## Beschreibung

Die Erfindung betrifft eine Anordnung zur ferngesteuerten Freisetzung von Wirkstoffen gemäß der Gattung der Patentansprüche.

In verschiedenen Gebieten der Technik und der Biomedizin, insbesondere in der Medizintechnik, besteht die Aufgabe, an Stellen, die nicht unmittelbar zugänglich sind (z. B. im menschlichen Verdauungstrakt), aus einem Vorratsbehälter, bspw. einer Kapsel, Stoffe ferngesteuert zu beliebig wählbaren Zeiten freizusetzen, ohne dass eine Verbindung durch Schläuche, elektrische Leitungen oder dergleichen zum Vorratsbehälter verwendet wird.
Speziell für die ferngesteuerte Freisetzung von Wirkstoffen im Verdauungstrakt sind aus dem Stand der Technik verschiedene Mittel und Verfahren bekannt, bei denen Kapseln mit relativ kompliziertem Aufbau verwendet werden. Typische Beispiele sind eine so genannte RF-Kapsel [S. P. Eriksen et al., J. Pharmaceutical Sciences 50 (1961) S. 151], eine Dünndarm-Kapsel [A. Hemmati, Dtsch. Med. Wschr. 93 (1968) S. 1468], eine HF-Kapsel [B. Hugemann und O. Schuster, Deutsche Patentschrift DE 29 28 477 (1979)], eine InteliSite®-Kapsel [A. F. Parr et al., Pharmaceutical Research 16 (1999) S. 266] und ein Marker für Darm-Diagnostik und -Therapie [W. Andrä und M. Wendt, DE 197 45 890 (1999)]. Weitere Kapseln sind in den US-Patentschriften 4,239,040 und 5,279,607 beschrieben.
Alle bekannten Ausführungsformen der Kapseln besitzen mindestens einen der folgenden Nachteile:
1. Einige Kapseln enthalten nicht-biokompatible Teile. In einigen Formen sind Batterien enthalten, andere enthalten unterschiedliche Metalle in Form von Federkörpern oder Heizdrähten oder als elektrische Leiter. Daher müssen diese Kapseln mit einer dichten biokompatiblen Hülle geschützt sein. Im Falle einer unbeabsichtigten Beschädigung dieser Hülle besteht die Gefahr, dass der toxisch wirkende Inhalt mit Körpergewebe in Berührung kommt.
2. Andere Kapseln besitzen eine starre Form und können im Magen-Darm-Trakt nicht aufgelöst werden. Daher ist vor dem Verschlucken zu prüfen, dass keine Einengungen des Verdauungstraktes vorhanden sind, an denen diese starren Kapseln hängen bleiben können, da dann die Entfernung möglicherweise nur durch einen invasiven Eingriff möglich ist.
3. Das Wirkprinzip einiger Kapseln besteht darin, dass durch ein magnetisches Wechselfeld in einem elektrisch leitfähigen Teil Wirbelströme oder magnetische Verluste induziert werden, die zur Erwärmung und, bei Erreichen einer Schwellentemperatur, zum Öffnen eines Verschlusses führen. Das außerhalb des menschlichen Körpers erzeugte magnetische Wechselfeld wirkt aber nicht nur in dem für die Erwärmung vorgesehenen Teilvolumen der Kapsel, sondern auch im elektrisch leitfähigen Körpergewebe. Damit dort keine unzulässige Erwärmung durch Wirbelströme eintritt, muss das Produkt aus der Amplitude H und der Frequenz f des Magnetfeldes unterhalb eines Maximalwertes (im Folgenden "Brezovich-Limits" genannt) bleiben, der zum Beispiel für die Ganzkörper-Exposition mit 4,85·10⁸ A/(s·m) angegeben worden ist [I. A. Brezovich, Medical Physics Monograph 16 (1988), S. 82]. Dadurch wird die in Wärme umsetzbare Leistung des Wechselfeldes begrenzt und die sichere Funktion der Freisetzung eingeengt.

Der Erfindung liegt die Aufgabe zu Grunde, eine Anordnung zur ferngesteuerten Freisetzung von Wirkstoffen zu schaffen, welche die vorgenannten Nachteile vermeidet.

Diese Aufgabe wird erfindungsgemäß durch die kennzeichnenden Merkmale des ersten Patentanspruchs gelöst. An Stelle von magnetischen oder elektrischen Verlusten in den wechselfeldexponierten Kapselteilen werden die Reibungsverluste benutzt, die bei der Bewegung eines magnetischen Körpers in einer Flüssigkeit entstehen. Der magnetische Körper kann sich dabei direkt in einem Hohlraum befinden, der mit Wirkstoff gefüllt ist, oder er ist in einer mit einer geeigneten Flüssigkeit gefüllten Kapsel gelagert, die ihrerseits in dem Wirkstoff im Hohlraum gelagert ist. Weitere Verbesserungen erfährt die Erfindung durch die Merkmale der Unteransprüche. Der als Rotor ausgebildete Körper wird entweder durch ein sich drehendes Magnetfeld gedreht, zu dessen Erzeugung es mindestens zweier Spulen oder Spulenpaare bedarf, oder aber durch ein selbstgetriggertes Wechselfeld, wozu nur eine Spule erforderlich ist.

Die Erfindung wird nachstehend an Hand der schematischen Zeichnung zweier Ausführungsbeispiele näher erläutert. Es zeigen:
- Fig. 1: eine zur erfindungsgemäßen Anordnung gehörende Kapsel im Längsschnitt,
- Fig. 2: die Spulen mit einer zugehörigen Kapsel einer erfindungsgemäßen Anordnung in perspektivischer Darstellung,
- Fig. 3: die erfindungsgemäße Anordnung mit nur einer Spule in perspektivischer Darstellung,
- Fig. 4a: die zeitliche Abfolge der z-Komponente des Magnetfeldes eines Rotors und
- Fig. 4b: die zeitliche Abfolge der Stromimpulse und eines magnetischen Wechselfeldes bei Verwendung nur einer Spule.

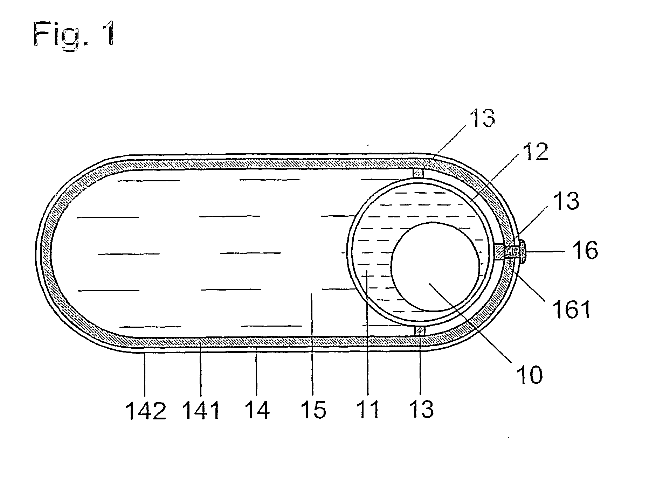

In Fig. 1 ist ein kugelförmiger Rotor 10, der einen Durchmesser (∅) von 5 mm aufweist, in einer wasserfreien Flüssigkeit 11 geringer Viskosität frei schwimmend gelagert. Der Rotor 10, als dauermagnetischer Körper, besteht aus ca. 50 Vol% Fe₃O₄ mit einer Koerzitiv-Feldstärke H_{C} ≈ 30 kA/m und aus ca. 50 Vol% Gelatine. An Stelle von Fe₃O₄ kann auch γ - Fe₂O₃ und statt Gelatine kann eine andere nichttoxische, wasserlösliche Substanz (z. B. Zucker) verwendet werden. Bei der Flüssigkeit 11 handelt es sich um ein Siliconöl mit einer Viskosität von η = (1...10) · 10⁻³ N·s/m². An Stelle von Siliconöl ist es möglich, Speiseöl mit niedriger Viskosität zu verwenden. Die Flüssigkeit 11 befindet sich in einer Hohlkugel 12 aus Hartgelatine oder einer anderen nichttoxischen, wasserlöslichen Substanz, bspw. Zucker, mit einem Innendurchmesser ∅ᵢ = 7,6 mm und einem Außendurchmesser ∅ₐ = 8,0 mm. Die Hohlkugel 12, die aus zwei oder mehreren dicht verbundenen Teilen hergestellt sein kann, ist über Halter 13 mit einer ovalen oder länglichen Kapsel 14 fest verbunden und in dieser gehaltert. Die Kapsel ist im Übrigen mit einem geeigneten Wirkstoff 15 gefüllt. Die Halter 13, von denen mindestens einer vorhanden sein muss, bestehen aus einer Mischung von ca. 50 Vol% Gelatine und ca. 50 Vol% Graphit; in dieser Zusammensetzung sind sie geeignet, auch als Wärmebrücken zu dienen. An Stelle von Gelatine kann wieder eine andere geeignete Substanz und an Stelle von Graphit kann bspw. Silizium-Pulver als nichttoxischer Stoff mit hoher Wärmeleitfähigkeit eingesetzt werden. Die Kapsel 14, bspw. der genormten Größe 00, deren ∅ 8,5 mm und. Länge 28 mm betragen, besteht in ihrem wesentlichen, inneren Teil 141 aus Hartgelatine, die außen mit einem dünnen Überzug 142, bspw. aus Polyäthylen, zum Schutz gegen eine Auflösung durch eine aggressive Flüssigkeit, bspw. Darmflüssigkeit, versehen ist. Die Kapsel 14 hat eine Öffnung 161, die durch einen Verschluss 16 aus Hartparaffin oder einem tierischen bzw. pflanzlichen Wachs mit einem Schmelzpunkt von 50 bis 55 °C verschlossen ist. Ist der Wirkstoff 15 wasserhaltig, muss die Innenwand der Kapsel 14 ebenfalls mit einem Überzug versehen sein, der vorzugsweise aus dem Material des Schmelzverschlusses besteht.

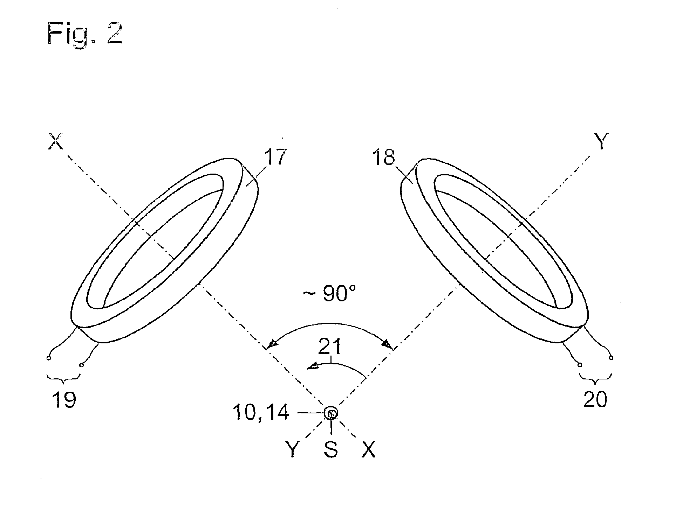

Die in Fig. 1 beschriebene Kapsel 14 bzw. der in ihr befindliche dauermagnetische Körper 10 befindet sich gemäß Fig. 2 im Schnittpunkt S oder in der Nähe des Schnittpunktes der Achsen X-X, Y-Y zweier Spulen 17, 18, der von beiden Spulen 17, 18 etwa 15 cm entfernt ist, wobei diese Spulen Durchmesser von ca. 22 cm haben und ihre Achsen zumindest nahezu senkrecht aufeinander stehen. Die Anschlüsse beider Spulen 17, 18 sind mit 19, 20 bezeichnet. Die Spulen 17, 18 werden von Wechselstrom mit einer Phasenverschiebung zwischen den beiden Spulen 17, 18 von etwa 90° durchflossen, der am Ort der Kapsel 14 ein rotierendes Magnetfeld mit der Amplitude H von 0,3 ... 3 kA/m bei einer Frequenz f von 1 ... 10 kHz erzeugt.
Durch das rotierende Magnetfeld H wird der dauermagnetische Körper 10 mit dem magnetischen Moment m zur Rotation in dem durch einen Pfeil 21 angezeigten Drehsinn gezwungen. Bspw. dreht sich das Magnetfeld H mit einer vorgegebenen festen Drehzahl von 1000 Hz ≙ 60000 Umdrehungen/min. Die dabei entstehende Reibung zwischen dem Rotor 10 und der Flüssigkeit 11 erwärmt diese und über die Hohlkugel 12 sowie den entsprechenden Halter 13 den Verschluss 16, so dass dieser schmiltzt und die Wirkflüssigkeit 15 aus der Kapsel 14 austreten kann. Damit möglichst große Reibungsverluste zu Stande kommen, also eine möglichst starke Erwärmung eintritt, ist die Oberfläche des dauermagnetischen Rotors 10 vorzugsweise mit einer genügend großen Anzahl von Reibungsansätzen versehen, welche die Form von Warzen oder Flügeln haben können. Die Amplitude des Magnetfeldes richtet sich nach der gewählten Frequenz und nach der Viskosität der Flüssigkeit 11, in der sich der Rotor 10 befindet. Bei Speiseöl mit einer Viskosität von η = 0,04 N.s/m² und bei einer gewählten Frequenz von f = 500 Hz muss die Feldamplitude größer als ca. 8 kA/m sein, um eine Temperaturerhöhung von mehr als 10 K zu erreichen.

Durch die erfindungsgemäße Anordnung wird der oben unter 1. genannte Nachteil überwunden, indem der dauermagnetische Körper 10 aus Magnetit oder einem anderen als Lebensmittelfarbstoff zugelassenen magnetischen Eisenoxid hergestellt wird. Als Flüssigkeit kann Speiseöl oder eine andere nichttoxische Flüssigkeit verwendet werden. Kapselhüllen aus nichttoxischem Material sind bereits im medizinischen Einsatz.
Der oben unter 2. genannte Nachteil wird dadurch vermieden, dass alle Teile der Kapsel 14 ferngesteuert aus einer starren Form in eine Form überführt werden, die Einengungen des Verdauungstraktes passieren kann. Das ist bei dem dauermagnetischen Körper 10 dadurch möglich, dass er aus einem magnetischen Pulver besteht, das durch ein nichttoxisches Bindemittel, z. B. darmverträgliches Wachs oder darmverträgliche Gelatine, zusammengehalten wird. Bei Erwärmung der Flüssigkeit 11 durch Reibung wird nicht nur der temperatursensible Verschluss 16 der Kapsel 14 geöffnet und der Wirkstoff 15 freigegeben. Der dauermagnetische Körper 10 wird ebenfalls in kleinere Teile (Pulver und deformierbares Wachs o. dgl.) überführt, die Stenosen des Darmes passieren können. Die Auflösung der restlichen Kapselteile wird dadurch gesichert, dass diese aus einem wasserlöslichen Material (z. B. Hartgelatine) hergestellt sind, das nur an denjenigen Oberflächen durch eine wasserunlösliche Hülle (z. B. aus Polyäthylenfolie) geschützt ist, welche vor dem Öffnen des temperatursensiblen Verschlusses 16 mit Wasser oder Körpergewebe in Berührung sind. Nach dem Öffnen des temperatursensiblen Verschlusses 16 und dem Zerfall des Rotors 10 kann die Darmflüssigkeit oder eine andere Flüssigkeit in die Kapsel 14 eindringen und deren Auflösung von innen bewirken.
Wegen des erfindungsgemäßen Wirkmechanismus der Wärmeerzeugung kann der oben unter 3. genannte Nachteil ebenfalls vermieden werden. Zur Begründung wird hierzu die Wirkungsweise einer erfindungsgemäßen Anordnung einer dem Stand der Technik entsprechenden Anordnung gegenübergestellt. Im Folgenden wird unter a) durch Ummagnetisierungsverluste einer Kugel und unter b) durch die Reibung einer dauermagnetischen Kugel in einer Flüssigkeit geeigneter Viskosität Wärme erzeugt.
a) Die Kugel von 4 mm Radius besteht aus handelsüblichem weichmagnetischen Magnetit-Pulver, dessen Packungsdichte hinsichtlich maximaler Leistungsdichte optimiert ist. Sie wird in einem magnetischen Wechselfeld mit einer Frequenz von 80 kHz und einer einstellbaren Amplitude ummagnetisiert. Um das oben genannte "Brezovich-Limit" einzuhalten, darf dann die Amplitude des Wechselfeldes 6 kA/m nicht übersteigen. Dabei erreichen die Ummagnetisierungsverluste in der Kugel ca. 0,23 W. Im thermischen Gleichgewicht wird dadurch ein Temperaturanstieg um 7,5 K erreicht. Ein Magnetfeld von 6 kA/m bei einer Frequenz von 80 kHz innerhalb des menschlichen Körpers zu erzeugen, bedeutet allerdings einen erheblichen technischen Aufwand.
b) Aus Wachs und einem speziellen hartmagnetischen Magnetit-Pulver (Koerzitiv-Feldstärke: 35 kA/m) wird eine Kugel mit einem Radius von 2,5 mm mit einer Packungsdichte von 80 Vol% Magnetit gepresst. Sie wird innerhalb einer Polyäthylen-Hohlkugel von 3,8 mm Innenradius und 4,0 mm Außenradius in Wasser gelagert. Die Anordnung wird in einem Magnetfeld von ca. 800 kA/m aufinagnetisiert und hat danach ein remanentes magnetisches Moment von ca. 0,027 A·m². Bei der klinischen Anwendung erfolgt dieses Aufinagnetisieren außerhalb des Patienten vor dem Schlucken der Kapsel. Danach wird die innere Kugel durch ein magnetisches Drehfeld zur dauernden Rotation mit einer Frequenz von 6,3 kHz angeregt. Die Hohlkugel wird dabei gegen ein mögliches Rotieren festgehalten, was im klinischen Einsatz durch Einbau in eine Kapsel realisiert wird. Dabei entstehen in der Flüssigkeit Reibungsverluste von 2,4 W, also mehr als das Zehnfache des oben genannten Beispiels a). Der dadurch erreichte Temperaturanstieg beträgt im thermischen Gleichgewicht ca. 20 K. Um die viskose Reibung des Wassers zu überwinden, ist eine Amplitude des Drehfeldes von 1,9 kA/m erforderlich, wodurch das Produkt aus Amplitude und Frequenz bei 1,2·10⁷ A/(s·m) und damit um den Faktor 40 unterhalb des "Brezovich-Limits" liegt.

Im Fall b) wird also mit einer wesentlich niedrigeren Feldamplitude und einer niedrigeren Frequenz eine zehnfach größere Wärmeleistung als im Fall a) erzeugt. Da sie sich aber nicht nur auf das Flüssigkeitsvolumen, sondern auch auf den Rotor und die Hohlkugel verteilt, beträgt die praktisch erzeugte Temperaturerhöhung nur etwas mehr als das Doppelte gegenüber dem Fall a). Das Produkt aus Amplitude und Frequenz bleibt dabei weit unter dem "Brezovich-Limit".
Allerdings müssen, wie oben zu Fig. 1 beschrieben, zur Erzeugung eines Drehfeldes zwei Spulen oder Spulenpaare mit Wechselstrom betrieben werden, die eine Phasendifferenz von etwa 90° besitzen. Die genannte Frequenz von 6,3 kHz und die Feldamplitude von 1,9 kA/m sind jedoch mit moderatem technischen Aufwand zu realisieren.

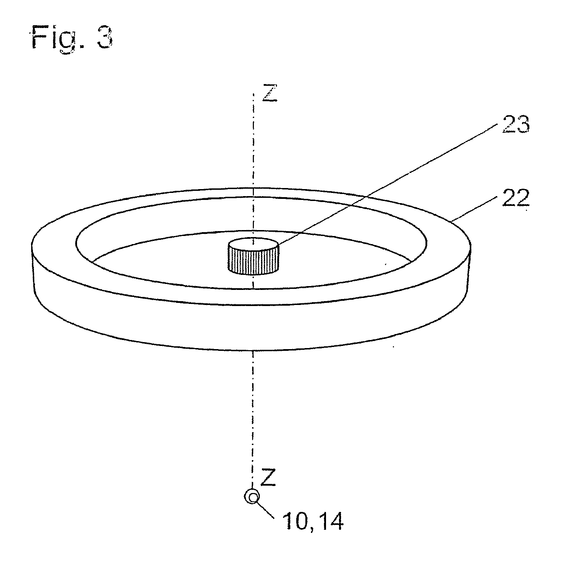

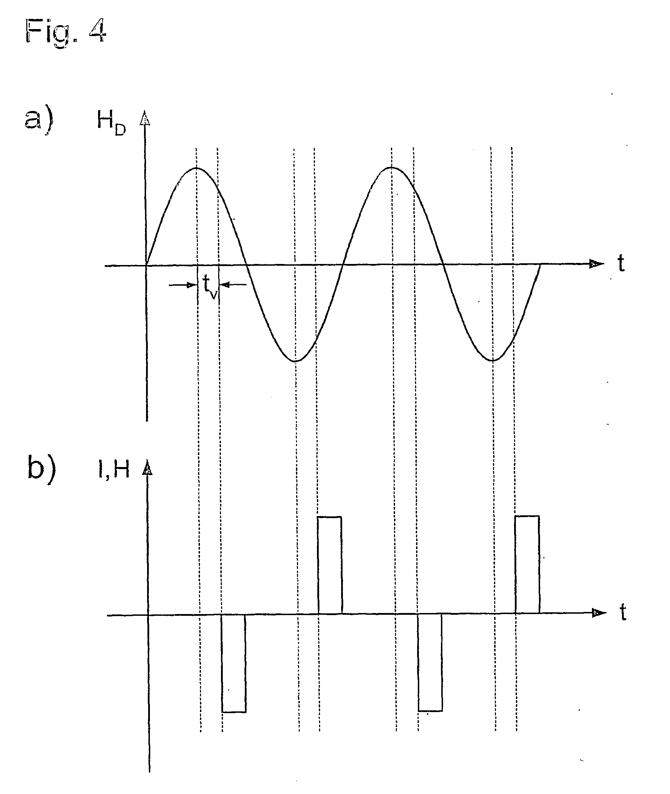

In Fig. 3 ist ein dauermagnetischer Körper (Rotor) 10 mit einem magnetischen Moment m in einem Abstand von etwa 10 cm von einer ringförmigen, wechselstromdurchflossenen Einzelspule 22 auf deren Achse Z-Z angeordnet. Die Spule 22 hat selbst einen mittleren Durchmesser von 15 cm. Amplitude und Frequenz des Wechselfeldes H am Ort des Rotors 10 sind wie zu Fig. 2 beschrieben. Im Zentrum der Spule 22 ist ein Sensor 23 angeordnet, der bspw. magnetoresistiv arbeitet und der die Komponenten des magnetischen Rotorfeldes detektiert. Durch die Spule 22 fließt ein Impulsstrom I mit einem Tastverhältnis von etwa 1:10, d. h. der Strom fließt durch die Spule 22 nur während 10 % der Zeit. Für die Dauer der Strompause wird der Sensor eingeschaltet. Die Extrema des Sensorsignals, das bspw. die z-Komponente des vom Rotor 10 ausgehenden Magnetfeldes H_{D} (Fig. 4a) anzeigt, werden benutzt, um mit einstellbarer Verzögerungszeit tᵥ die Stromimpulse zu. triggern (Fig. 4b). Durch Kontrolle der Sensorsignale wird die Verzögerungszeit, die sich aus einem Vergleich der Figuren 4a und 4b ergibt, optimiert, d. h. die Drehbewegung des Rotors 10 wird ungedämpft aufrecht erhalten.
Die durch das selbst getriggerte Wechselfeld verringerte Anzahl der Spulen verringert den Aufwand insgesamt. Die Triggerung wird nach Messung des Magnetfeldes H_{D} ausgelöst, das von dem magnetischen Moment m des dauermagnetischen Körpers 10 ausgeht. H ist das von der Spule 22 erzeugte impulsförmige Magnetfeld. Zwischen den periodischen Zeitläufen der Felder H und H_{D} besteht eine Zeitverschiebung, die in Fig. 4 mit tᵥ bezeichnet ist. Das impulsförmige Magnetfeld H wird eingeschaltet, wenn die Zeit tᵥ nach Erreichen des Maximalwertes bzw. des Minimalwertes des Feldes H_{D} verstrichen ist. Überschreitet diese Zeitverschiebung tᵥ einen vorgegebenen Wert, so ist das Drehmoment, das vom Feld H auf den Rotor 10 ausgeübt wird, hinreichend groß, um die Drehbewegung des Rotors 10 aufrecht zu erhalten. Damit möglichst große Reibungsverluste zu Stande kommen, kann auch bei diesem Ausführungsbeispiel die Form des rotierenden Körpers so gestaltet werden, dass in der Lagerungsflüssigkeit Turbulenzen entstehen, die höhere Reibungsverluste und damit größere Erwärmungen zur Folge haben als turbulenzfreie Strömungen bei gleicher Rotationsfrequenz. Das kann bspw. durch Anbringen von warzen- oder flügelförmigen Ansätzen an einen kugelförmigen Körper geschehen.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 10: magnetischer Körper (Rotor)
- 11: Flüssigkeit
- 12: Hohlkugel
- 13: Halter
- 14: Kapsel
- 15: Wirkstoff
- 16: Verschluss
- 17,18: Spulen
- 19, 20: Anschlüsse
- 21: Pfeil (Drehsinn)
- 22: Einzelspule
- 23: Sensor
- 141: innerer Teil
- 142: Überzug
- 161: Öffnung
- X-X, Y-Y, Z-Z: Achsen

## Patentansprüche

1. Anordnung zur ferngesteuerten Freisetzung von Wirkstoffen (15), die sich mit einem in einer Flüssigkeit (11) gelagerten magnetischen Körper (10) in einem Hohlraum befinden und unter dem Einfluss einer Erwärmung freigesetzt werden, **dadurch gekennzeichnet, dass** der magnetische Körper (10) in diesem Hohlraum durch mindestens ein von außen angelegtes Magnetfeld zur Rotation gebracht wird und durch seine Reibung in der Flüssigkeit (11) Wärme erzeugt, die zur Freisetzung des Wirkstoffs (15) benutzt wird.

2. Anordnung gemäß Anspruch 1, **dadurch gekennzeichnet, dass** der magnetische Körper (10) in einer mit einer Flüssigkeit (11) gefüllten Kapsel (14) und diese in dem Hohlraum angeordnet ist.

3. Anordnung gemäß Anspruch 1, **dadurch gekennzeichnet, dass** der Körper (10) mit Reibungsansätzen versehen ist.

4. Anordnung gemäß Anspruch 1, **dadurch gekennzeichnet, dass** der Körper (10) aus einem dauermagnetischen Material besteht.

5. Anordnung gemäß Anspruch 1, **dadurch gekennzeichnet, dass** der Körper (10) einem magnetischen Wechselfeld ausgesetzt ist, das von mindestens zwei stromdurchflossenen Spulen erzeugt wird.

6. Anordnung gemäß Anspruch 1, **dadurch gekennzeichnet, dass** der Körper (10) einem getriggerten magnetischen Wechselfeld ausgesetzt ist, das durch eine stromdurchflossene Spule erzeugt wird.

## Claims

1. An arrangement for the remote-controlled release of active ingredients (15) which are located with a magnetic body (10) in a cavity and are released under the influence of heat, the magnetic body (10) being embeded in a fluide, **characterised in that** said magnetic body (10) in said cavity is stimulated to rotate by means of a magnetic field that is applied from the outside and the friction of said magnetic body in the fluid (11) produces heat which is used for releasing the active ingredient (15).

2. The arrangement of claim 1, wherein said magnetic body (10) is arranged in a capsule (14) filled with a fluid (11) and said capsule is integrated into the cavity.

3. The arrangement of claim 1 wherein friction-increasing pieces are attached to said body (10).

4. The arrangement of claim 1, wherein said body (10) consists of a permanent-magnetic material.

5. The arrangement of claim 1, wherein said body (10) is exposed to an alternating magnetic field that is generated by at least two current-carrying coils.

6. The arrangement of claim 1, wherein said body (10) is exposed to a triggered alternating magnetic field that is generated by one current-carrying coil.

## Revendications

1. La configuration d'un dispositif destiné pour la libération télécommandée de substances actives (15) situées dans une cavité d'un corps magnétique (10) lui-même logé dans un liquide (11), et libérées par échauffement, est **caractérisée en ce que** le corps magnétique (10) se trouvant dans cette cavité est mis en rotation par application depuis l'extérieur d'au moins un champs magnétique et **en ce que** c'est son frottement dans le liquide (11) qui produit la chaleur utilisée pour la libération de la substance active (15).

2. La configuration suivant la revendication 1 est **caractérisée en ce que** le corps magnétique (10) est disposé dans une capsule (14) remplie d'un liquide (11) et logée dans une cavité.

3. La configuration suivant la revendication 1 est **caractérisée en ce que** le corps (10) est doté de points de frottement.

4. La configuration suivant la revendication 1 est **caractérisée en ce que** le corps (10) est composé d'un matériau magnétisé de manière permanente.

5. La configuration suivant la revendication 1 est **caractérisée en ce que** le corps (10) est soumis à un champ alternatif magnétique généré par au moins deux bobines parcourues par un courant électrique.

6. La configuration suivant la revendication 1 est **caractérisée en ce que** le corps (10) est soumis à un champ alternatif magnétique intermittent (mode trigger) généré par une bobine parcourue par un courant électrique.
